# EUROPEAN PATENT SPECIFICATION

(11) **EP 1 307 438 B1**
(45) Date of publication and mention of the grant of the patent: **07.09.2005**
(21) Application number: 01962875.9
(22) Date of filing: 26.07.2001
(51) Int. Cl.: C07D 311/08, A61K 31/37, A61P 7/00, A61P 39/00

(54) **NOVEL COUMARIN DERIVATIVES AND THE SALTS THEREOF, A PROCESS FOR THE PREPARATION THEREOF AND THEIR USE IN THE PHARMACEUTICAL FIELD**
CUMARIN-DERIVATE UND IHRE SALZE, VERFAHREN ZU IHRER HERSTELLUNG UND IHRE PHARMAZEUTISCHE VERWENDUNG
NOUVEAUX DERIVES DE COUMARINE ET SELS CORRESPONDANTS, PROCEDE DE PREPARATION CORRESPONDANT ET LEUR UTILISATION DANS LE DOMAINE PHARMACEUTIQUE

(30) Priority: 31.07.2000 IT PD000193
(43) Date of publication of application: 07.05.2003
(73) Proprietor: BAUSCH & LOMB INCORPORATED, Rochester, NY 14604-2701 (US)
(72) Inventor: PROSDOCIMI, Marco, I-35122 Padova (IT); MENON, Giampaolo, I-35041 Battaglia Terme (IT); MONASTRA, Giovanni, I-35132 Padova (IT); GALBIATI, Enrico, I-35031 Abano Terme (IT); FINESSO, Mario, I-35036 Montegrotto Terme (IT)
(74) Representative: Minoja, Fabrizio
(86) International application number: PCT/EP2001/008642
(87) International publication number: WO 2002/010148

(56) References cited:
- EP-A- 0 655 242
- WO-A-92/22545
- WO-A-95/00142
- BE-A- 621 327
- DE-B- 1 246 754
- FR-A- 1 451 667
- US-A- 3 243 441
- US-A- 3 259 635
- BING H W ET AL: "Specific inhibition of cyclic AMP-dependent protein kinase by warangalone and robustic acid" PHYTOCHEMISTRY, PERGAMON PRESS, GB, vol. 44, no. 5, March 1997 (1997-03), pages 787-796, XP004292922 ISSN: 0031-9422
- SQUADRITO F ET AL: "THE EFFECT OF CLORICROMENE, A COUMARINE DERIVATIVE, ON LEUKOCYTE ACCUMULATION, MYOCARDIAL NECROSIS AND TNF-ALPHA PRODUCTION IN MYOCARDIAL ISCHAEMIA-REPERFUSION INJURY" LIFE SCIENCES, PERGAMON PRESS, OXFORD, GB, vol. 53, no. 4, 1993, pages 341-355, XP001029718 ISSN: 0024-3205
- ZHOU P ET AL: "Coumarins and bicoumarin from Ferula sumbul: anti-HIV activity and inhibition of cytokine release" PHYTOCHEMISTRY, PERGAMON PRESS, GB, vol. 53, no. 6, 8 March 2000 (2000-03-08), pages 689-697, XP004291361 ISSN: 0031-9422

## Description

The present invention relates to novel coumarin derivatives the salts of said compounds, particularly those with pharmaceutically acceptable bases or acids, and the processes for the preparation thereof.

The novel coumarin derivatives and the salts according to the present invention have interesting pharmacological properties and may therefore be used to advantage to treat major pathologies such as peripheral ischaemia and organ ischaemia, electrical alterations of the myocardium and other organs resulting from the release of pro-inflammatory molecules (TNF, IL-1, NO, etc), peripheral and cerebral vasculopathies, angina-type disorders, hypercholesterolaemia, systemic infections such as sepsis, allergic pathologies such as asthma, rhinitis, eczema, dermatitis, such as antithrombotics and antihypertensives. The present invention also includes pharmaceutical preparations containing one or more of said derivatives or their salts in the form of capsules, tablets, injectable solutions, sprays and controlled release systems, creams, gels and transdermal systems.

### BACKGROUND OF THE INVENTION

Coumarins include a large class of phenol substances that are to be found in plants, and are constituted by a benzene ring and an α-pyrone ring fused together. To date, at least 1,300 coumarins have been identified, mainly as metabolites of green plants, in fungi and in bacteria.

Cloricromene (commercial name Proendotel) belongs to a family of coumarins and is prepared by the process described in U.S. patents No.s 4,296,039 and 4,452,811.

Besides its known coronary vasodilatory and anti-arrhythmic properties (US 4,349,566) and anti-platelet aggregation properties (US 4,362,741), it has been seen that cloricromene is able to inhibit many of the cellular functions of polymorphonuclear leukocytes (Bertocchi et al.: Arch. Pharmacol. 1989, 339, 697-703; Gresele et al.: Biochem. Pharmacol. 1993, 45, 123-130) and may have positive effects on biochemical interactions between different cell species, in particular between platelets and polymorphonuclear leukocytes (Zatta et al.: Eur. J. Pharmacol. 1991, 198, 97-100), that are known to be relevant in thrombotic and ischaemic states.

Cloricromene also has a documented effect in models of ischaemia and reperfusion in various organs, in which inflammatory-type cytokines are also involved, such as TNF (Squadrito et al.: Life Sciences 1993, 53, 341-355), and its activity is also known in the treatment of pathologies linked with vasodilatory processes and tissue damage characterised by an increased production of nitrogen oxide (patent by the Applicant No. IT 1265665), such as, for example, pulmonary inflammation, oedema, erythema, dermatitis, psoriasis, skin ulcers, arthritis, rheumatoid arthritis and other autoimmune disorders, hypotensive shock, septic shock, hypovolemic shock, vasculitis such as inflammations consequent on thrombo-phlebitis, ulcerative colitis.

### DETAILED DESCRIPTION OF THE INVENTION

In view of these known properties of Cloricromene's and since cytokines are known to be strong pro-inflammatory agents, a series of compounds have been prepared and tested for their activity *in vitro* and *in vivo* on the synthesis of cytokines such as TNF, as well as for their action on other cellular phenomena, such as platelet aggregation and the production of free radicals, as well as their action in *in vivo* models of inflammation. Among the drugs currently used in inflammatory processes, the non-steroid compounds are known for their poor, or lack of, ability to inhibit the release of inflammatory cytokines, that are, on the contrary, inhibited by steroid-.type compounds. Since these compounds have various toxic activities, the availability of non-steroid products specifically active on cytokine synthesis is particularly useful in developing innovative therapies.

The experimental results obtained have demonstrated that the compounds that are the subject of the present invention have activities that are better than and different from those of cloricromene. In particular, it was observed that some compounds have stronger actions than cloricromene on the synthesis of TNF, a known inflammatory cytokine, and they are not active in the process of platelet aggregation and the release of free radicals.

This increased selectivity of action makes the compounds of the present invention therapeutically advantageous.

Furthermore, *in vivo* characterisation has indicated that the compounds of the invention have lower acute toxicity than cloricromene, as demonstrated by the fact that toxic or lethal effects are observed only at higher dosages.

Therefore, due to the strict relationship between the free compounds and the salts of the present invention, wherever feasible, all that is indicated hereafter with regard to the free substances will be true also of their salts.

Compounds of the present invention are the following:

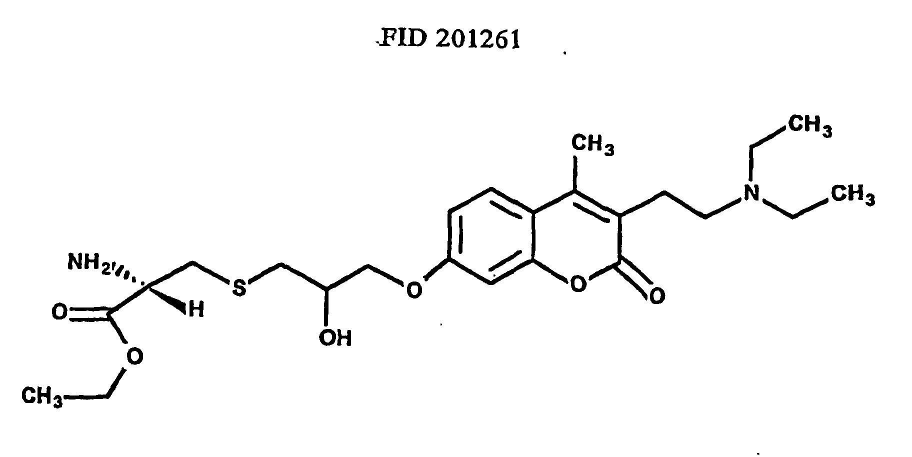

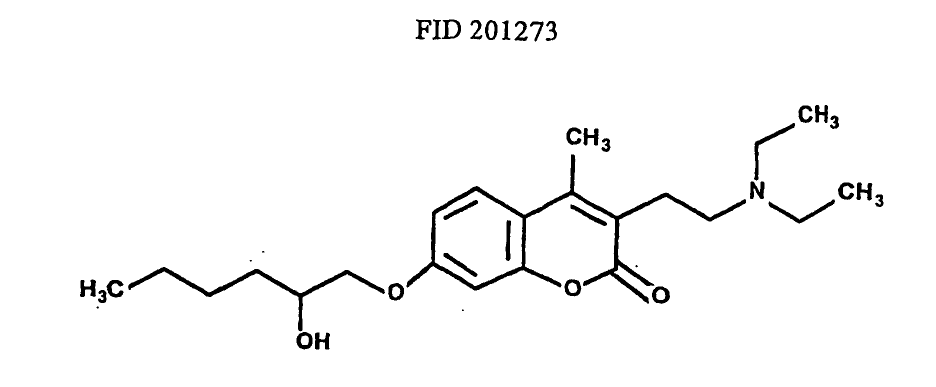

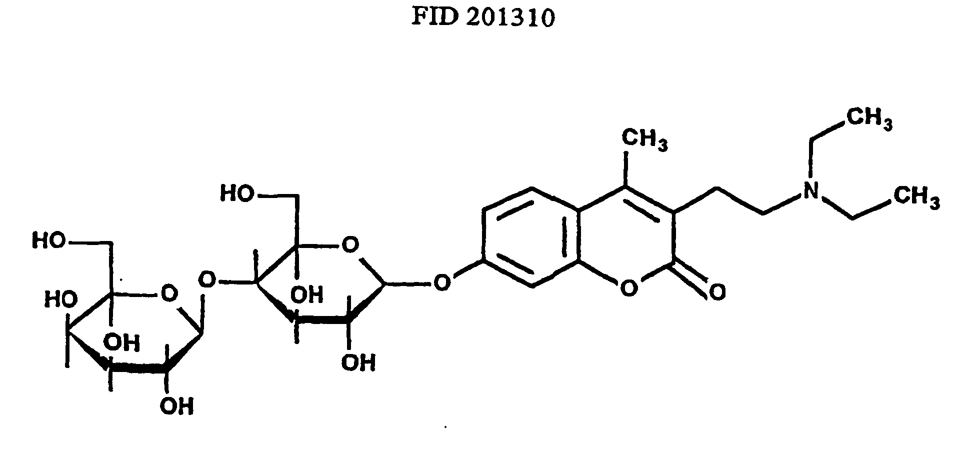

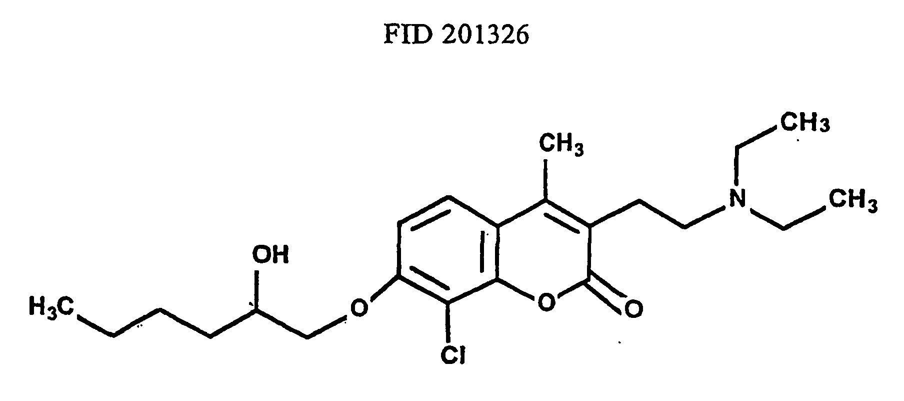

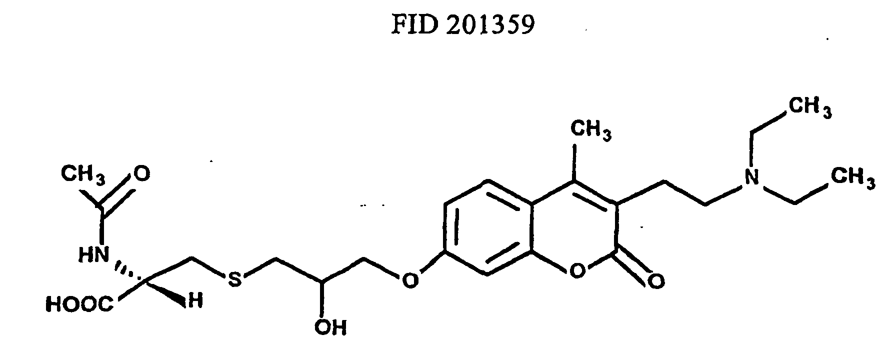

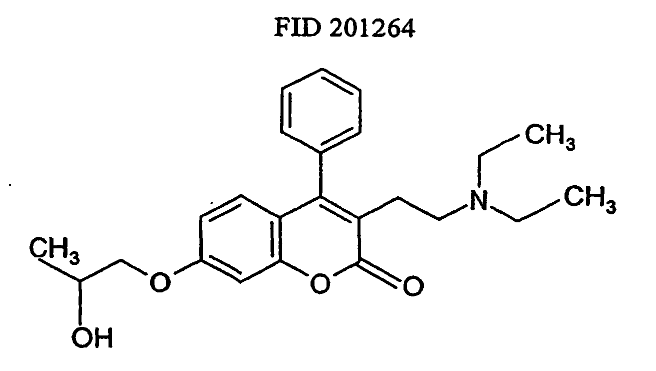

These were tested in various *in vitro* and *in vivo* models:
- inhibition of TNF release after stimulation with LPS *in vitro*
- inhibition of TNF release after stimulation with LPS *in vivo*
- reduced inflammation in carrageenin-induced oedema in rat paw
- inhibition of nitrite-nitrate release in rat plasma
- inhibition of superoxide anion formation induced by f-MLP in human whole blood
- inhibition of platelet aggregation in human whole blood
- acute toxicity after a single intravenous administration
- mutagenesis (mini-Ames test)

### Test 1 - inhibition of TNF release after stimulation with LPS in vitro

The test compound was added to the culture medium in a murine macrophage line (J774) or to whole blood anti-coagulated with heparin. The cells were then stimulated with bacterial lipopolysacccharide. After incubation at 37°C for a suitable length of time, the supernatant was removed from the culture and incubated with a line of murine fibroblasts (L929), sensitive to TNF. The quantity of TNF released after stimulation with LPS is measured by comparing mortality of the L929 cells with that of the controls. Table 1 reports for the single compounds the concentration (± standard error) able to inhibit fibroblast mortality (L929) by 50%.

**Table 1**

| **Compound** | **J774 CI**_{**50**} **(µM)** | **Human blood CI**_{**50**} **(µM)** |
|---|---|---|
| 201006 (Proendotel®) | 57.9 ± 20 | 78.6 ± 10 |
| 201261 | 25.4 | 9 |
| 201273 | 27.5 ± 5.3 | 40.4 ± 13.5 |
| 201326 | 10.5 ± 3.0 | 23 ± 2.0 |

### Test 2 - Inhibition of the release of TNF and IL-1β following stimulation with LPS in vivo

Conscious rats were injected with a dose of LPS, which is able to stimulate the release of TNF and IL-1β, having first received an intravenous administration (15 minutes beforehand) of the test compound at a dose of 0.5 mg/Kg or 2 mg/Kg. Blood levels of TNF and IL-1β were measured by the ELISA method on blood samples taken 75 minutes and 120 minutes after LPS respectively.

Table 2 below reports the percent of inhibition of each product compared to that of the controls treated with saline.

**Table 2**

| **Compound** | **Dose of 0.5 mg/Kg** | | **Dose of 2 mg/Kg** | |
|---|---|---|---|---|
| | % of inhibition of TNF | % of inhibition of IL-1β | % of inhibition of TNF | % of inhibition of IL-1β |
| 201006 (Proendotel) | 9 | 45 | 30 | 28 |
| 201273 | 72 | 67 | 84 | 39 |
| 201326 | 85 | 40 | 77 | 62 |

### Test 3 - Reduction in inflammation in carrageenin-induced oedema in rat paw

A model of acute inflammation induced by intraplantar injection of 1.5 mg of carrageenin in rat paw was used. Administration of the compounds (2 mg/Kg) was by the intravenous route 5 minutes before oedema was induced: 3 hours later the animals were sacrificed and their paws were weighed as an indication of inflammation. The control animals received saline instead of the test compounds. The weight of the paws of the control animals was taken to correspond to 100% on the inflammation index.

Results are shown in Table 3.

**Table 3**

| **Compound** | **% vs controls** |
|---|---|
| 201006 (Proendotel®) | 79 |
| 201261 | 86 |
| 201273 | 67 |
| 201326 | 58 |
| Indomethacin | 57 |
| Methylprednisolone | 56 |
| Saline solution | 100 |

### Test 4 - Inhibition of the release of nitrite-nitrate in rat plasma

The release of nitrite-nitrate was measured with Griess reagent in blood samples taken 8 hours after the administration of LPS (0.1 mg/Kg) i.v..

The animals were treated with the compounds by o.s. (25 mg/Kg) 30 minutes before and 3 hours after LPS. The control animals received saline instead of the test compounds. The values for the release of nitrite-nitrate were made to correspond to 100%. Results are reported in Table 4.

**Table 4**

| **Compound** | **% vs controls** |
|---|---|
| 201006 (Proendotel) | 99 |
| 201261 | 98 |
| 201273 | 76 |
| 201326 | 62 |
| Betamethasone | 20 |
| Saline solution | 100 |

### Test 5 - Inhibition of the formation of superoxide anions induced by f-MLP in human whole blood

Samples of whole blood diluted with PBS Cytochrome C were incubated for 20 minutes at 37°C with the test compounds or saline before adding the chemotactic agent f-MLP (0.1 µm/l) in the presence of cytochalasin B. After 20 minutes of activation with this agent, the samples were centrifuged and readings were taken of the supernatants with a spectrophotometer to assess the reduction in Cytochrome C. Table 5 reports the concentrations that are effective in inhibiting the loss of Cytochrome C by 50%.

**Table 5**

| **Compound** | **CI**_{**50**} **µM/l** |
|---|---|
| 201006 (Proendotel) | 52 |
| 201261 | 100 |
| 201273 | >100 |
| 201326 | >100 |
| SOD (unit/ml) | 0.5 |

### Test 6 - Inhibition of collagen-induced platelet aggregation in human whole blood

The aggregation event was analysed by counting the single non-aggregated platelets with a cell counter 5 minutes after adding collagen (1 µg/ml). In these working conditions, collagen induces platelet aggregation of over 80%. The test compounds or saline as control were preincubated for 1 minute before the collagen was added.

Table 6 reports the concentrations that inhibit platelet aggregation by 50% compared to saline.

**Table 6**

| **Compound** | **CI**_{**50**} **µM/l** |
|---|---|
| PG12 | 0.05 |
| 201006 (Proendotel) | 35.0 |
| 201261 | > 100 |
| 201273 | > 100 |
| 201326 | 78 |

### Test 7 - Toxicity after a single intravenous administration

Male CD-1 mice were used to assess the maximum non-lethal dose (MNLD - the highest dose at which no cases of mortality are observed) and the maximum tolerated dose (MTD - dose at which no evident or marked signs of toxicity or altered behaviour are observed). Results are shown in Table 7.

**Table 7**

| **Compound** | **DMT (mg/Kg)** | **DMNL (mg(Kg)** |
|---|---|---|
| 201006 (Proendotel) | 6.25 | 12.5 |
| 201261 | 50 | 200 |
| 201273 | 25 | 100 |
| 201326 | 6.25 | 50 |

### Test 8 - Mutagenesis (Mini-Ames test)

The Mini-Ames test is a version of the traditional Ames test which involves the incubation of bacterial cells on 35-mm dishes instead of 100-mm dishes, and it requires a smaller quantity of product while maintaining the reliability of the result.

The test is conducted on two strains of Salmonella typhimurium requiring histidine (TA 98 and TA 100) and two strains of Escherichia coli requiring tryptophan (WP2 pKM101 and WP2 uvrA pKM1091).

The bacterial cells were exposed to different concentrations of the test compounds in the presence and absence of a microsomal liver enzyme preparation, to reveal any possible metabolite activity.

Mutagenic activity was determined as the ability of the test compound to induce a significant increase in the number of mutant clones compared to those developed spontaneously in the cultures with the control vehicle.

The tested compound (201273, 201326) proved not to be mutagenic.

The compounds of the invention can be prepared by known methods (Claisen's condensation and rearrangement, Pechmann's condensation, Williamson's synthesis, Fischer's esterification). The coumarins are synthesised by condensation between a resorcin and a β-ketoester. They can then be alkylated by substituting the aromatic hydrogens by reaction with formaldehyde and an amine. Alternatively, it is possible to alkylate the free hydroxyl with an allyl and transpose it thermally onto the aromatic ring, or the hydroxyl can be alkylated with an alkylating agent or with a spacer (an alkyl dihalide or an alkyl halide substituted with an epoxy ring, an ester, an amine) that may then be further alkylated.

The synthesised product may undergo hydrogenation to reduce the double bond between positions 3 and 4, or the oxygen in position 2 can be substituted by reaction with Lawesson's reagent.

Some examples of the preparation of the coumarin derivatives according to the present invention are reported in the following:

### Example 1: preparation of the compound FID 201261

Ten grams of 3-diethylaminoethyl-4-methyl-7-hydroxy-coumarin (0.0363 mol MW 275.35) is salified with KOH in ethanol (1:1 mol/mol), the solvent is evaporated and the residue is redissolved in 2-butanone with 6.0 g of epibromohydrine (0.044 mol MW 136.98) by refluxing for 12 hours. The KBr salt is filtered off and the solvent is evaporated. The residue is redissolved in ethyl acetate and washed with 1N NaOH. The organic solution is dried, concentrated, precipitated with n-hexane and vacuum-dried. The residue is redissolved in water, added with 10.8 g of L-cysteine (0.0891 mol MW 121.16), the pH is adjusted to 9 and the mixture is left to react under stirring at 37°C. Twelve hours later, it is purified by silica gel chromatography with methylene chloride - methanol - ammonia 30% at a gradient of between 80-15-2 and 60-30-7

The clean fractions are concentrated, redissolved in ethanol saturated with HCl under stirring for one hour, evaporated and freeze-dried from water (yield 6.1 g MW 553.55).

### Example 2: preparation of the compound FID 201273

Five grams of 3-diethylaminoethyl-4-methyl-7-hydroxy-coumarin (0.0182 mol MW 275.35) is salified with KOH in ethanol (1:1 mol/mol). The solvent is evaporated and the residue is redissolved in 2-butanone with 9.1 g of epoxyhexane (0.091 mol MW 100.16) and refluxed for 48 hours. The KBr salt is filtered off and the solvent is evaporated. The residue is redissolved in ethyl acetate, washed with 1N NaOH, concentrated and purified by silica gel chromatography with an eluent of methylene chloride - methanol - ammonia, 30%, at a gradient of between 98-2-0.2 and 90-5-0.4.

The clean fractions are concentrated, redissolved in ethyl acetate and treated with HCl in ethanol until the Congo red indicator changes colour, concentrated and crystallised from acetone-hexane 2:1 (yield 3.1 g MW 411.97).

### Example 3: preparation of the compound FID 201310

Ten grams of lactose (0.0278 mol MW 360.32) is suspended in 51 g of acetic anhydride (0.5 mol MW 102.09) and added with 100 ml of pyridine anhydride, drop by drop. The mixture is reacted overnight, after which it is evaporated and purified by silica gel chromatography eluting with toluene-acetone 4:1. The pure fractions are evaporated and the resulting peracetyl-lactose is vacuum-dried, suspended in 120 ml of anhydrous N,N-dimethylformamide, added with 5.7 g of ammonium carbonate (0.059 mol MW 96.09) and reacted under stirring for 24 hours, then evaporated and purified on silica gel eluting with toluene-acetone 9:1.

The pure fractions are evaporated and the resulting hydroxyperacetyllactose is vacuum-dried, dissolved in 250 ml of anhydrous methylene chloride with 9.3 ml of trichloroacetonitrile (0.0642 mol MW 144.4) and treated with 0.5 g of sodium hydride (0.0214 mol MW 24), stirring for 3 hours. The mixture is concentrated and purified on silica gel eluting to a toluene-acetone gradient of between 9:1 and 7:3. The pure fractions are evaporated, the resulting peracetyl-lactose-trichloroacetamidate is vacuum-dried and reacted with 3.0 g of 3-diethylaminoethyl-4-methyl-7-hydroxy-coumarin in 100 ml of anhydrous methylene chloride, in the presence of activated molecular sieves. 3.2 g of boron trifluoride-ethyletherate (0.0109 mol MW 295.68) are added and the mixture is reacted for 2 hours, then filtered, washed with 1N NaOH, dried, concentrated and purified by silica gel chromatography with an eluent of methylene chloride - methanol - ammonia, 30%, at a gradient of between 90-5-0.4 and 80-20-0.4.

The clean fractions are concentrated, hydrolysed in 100 ml of 1N NaOH/tert-butanol 1:1, and neutralised with HCl. The pH is adjusted to 8 with ammonia and the mixture is extracted with chloroform/n-butanol 1:1.

The solvent is evaporated off and the residue is freeze-dried from water (yield 3.2 g MW 599.64).

### Example 4: preparation of the compound FID 201326

5.6 g of 3-diethylaminoethyl-4-methyl-7-hydroxy-8-chloro-coumarin (0.018 mol MW 309.8) is refluxed in toluene with 9.1 g of 1,2-epoxyhexane in the presence of 2 g of basic allumina super 1 for 24 hours. The solid matter is filtered off, washing with 1N NaOH. The mixture is concentrated and purified by silica gel chromatography with an eluent of methylene chloride - methanol - ammonia, 30%, at a gradient of between 98-2-0.2 and 90-5-0.4.

The clean fractions are concentrated, redissolved in ethyl acetate and treated with HCl in ethanol until the Congo red indicator changes colour, filtered and crystallised from acetone (yield 1.6 g MW 446.42).

### Example 5: preparation of the compound FID 201359

Ten grams of 3-diethylaminoethyl-4-methyl-7-hydroxy-coumarin (0.0363 mol MW 275.35) is salified with KOH in ethanol (1:1 mol/mol). The solvent is evaporated and the residue is refluxed in 2-butanone with 6.0 g of epibromhydrine (0.044 mol MW 136.98) for 12 hours. The KBr salt is filtered off and the solvent is evaporated. The residue is redissolved in ethyl acetate, washed with 1N NaOH, dried, concentrated, precipitated with n-hexane and vacuum-dried. The residue is redissolved in water and added with 14.5 g of N-acetyl L-cysteine (0.0891 mol MW 163.19), the pH is adjusted to 9 and the mixture is reacted under stirring at 37°C. Twelve hours later, it is freeze-dried and purified by silica gel chromatography with methylene chloride - methanol - ammonia, 30%, 80-25-5.

The clean fractions are concentrated and freeze-dried from HCl (yield 10.0 g MW 531.07).

### Example 6: preparation of FID 201264

30.3 g of 3-diethylaminoethyl-4-phenyl-7-hydroxy-coumarin (0,0802 mols, M.W. 337.86) (prepared as described in GB 1,013,053, example 1) are salified with KOH in ethanol (1 : 1 mols/mole); solvent is evaporated off, the residue is dissolved in 150 ml of DMSO and added with 26.5 g of 1-bromopropanol-2 (0.1605 mols, M.W. 165.07). After 10 days under stirring at 50°C, 300 ml of toluene and 150 ml of water are added. The organic phase is washed with 1M NaOH, then concentrated to dryness under vacuum. The residue is purified by silica gel chromatography with eluent CH2Cl2 - CH3OH - ammonia 30%, at a gradient of between 98 - 2 - 0.2 and 90 - 5 - 0.4. The clean fractions are concentrated to dryness, redissolved in ethyl acetate and treated with HCl in ethanol until the Congo red indicator has changed colour, then concentrated and freeze-dried from water (yield 13.5 g, M.W. 431.96).

All the products were characterised and their structures confirmed by NMR and FT-IR analysis.

## Claims

1. A coumarin derivatives selected from the group consisting of: 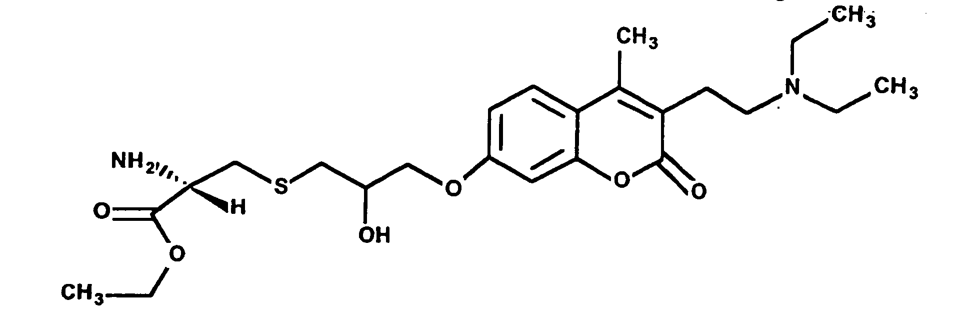 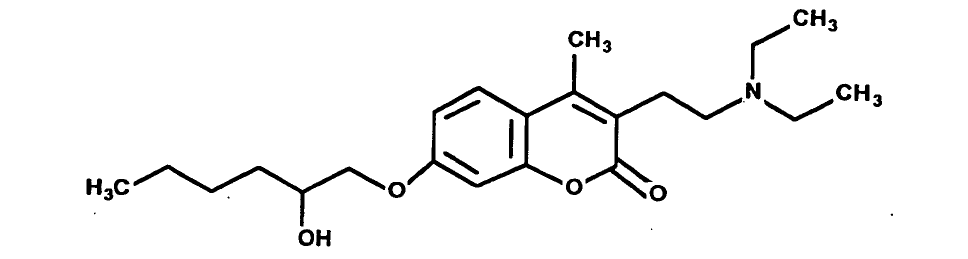 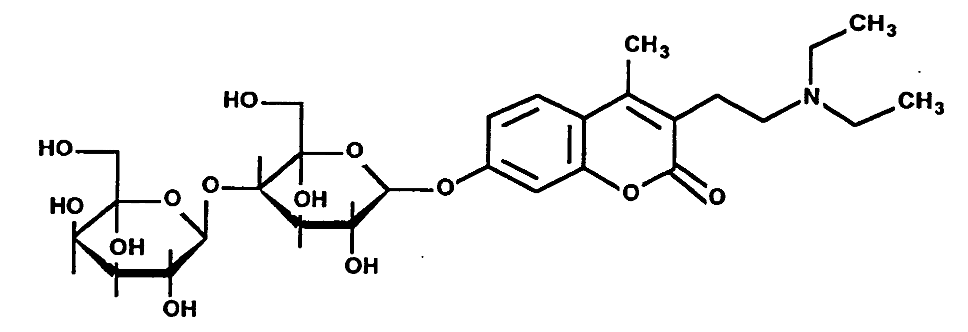 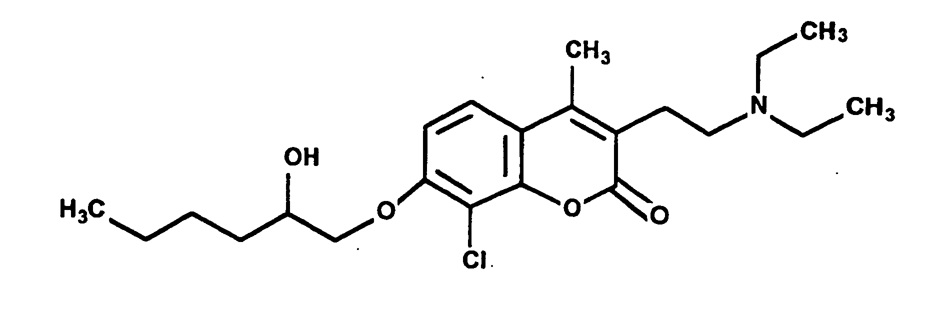 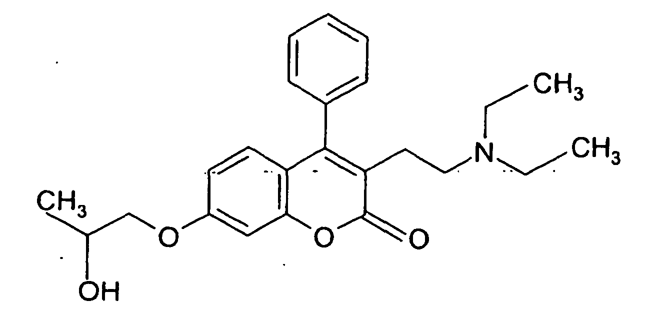 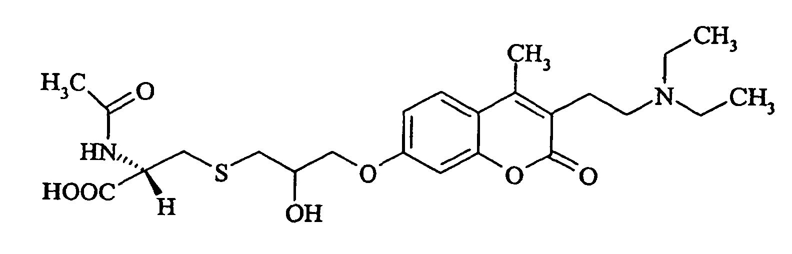 and the salts thereof with pharmaceutically acceptable acids or bases.

2. Pharmaceutical compositions containing a coumarine derivative as claimed in claim 1, in the form of capsules, tablets, injectable solutions, sprays, controlled release systems, creams, gels and transdermal systems.

3. Pharmaceutical compositions as claimed in claims 2, for the treatment of vascular (including those consequent on the release of pro-inflammatory molecules), dermatological and allergic pathologies, of hypercholesterolaemia and of systemic infections.

4. Pharmaceutical compositions as claimed in claims 2 and 3, for the treatment of peripheral vasculopathies, angina-type disorders and cerebral vasculopathies, peripheral ischaemia and ischaemia of organs.

5. Pharmaceutical compositions as claimed in claims 2 and 3, for the treatment of thrombosis and hypertension.

## Patentansprüche

1. Ein Coumarin-Derivat ausgewählt aus der Gruppe bestehend aus: 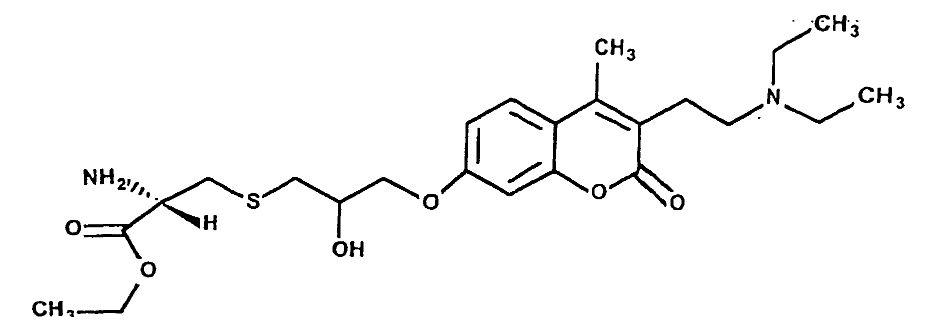 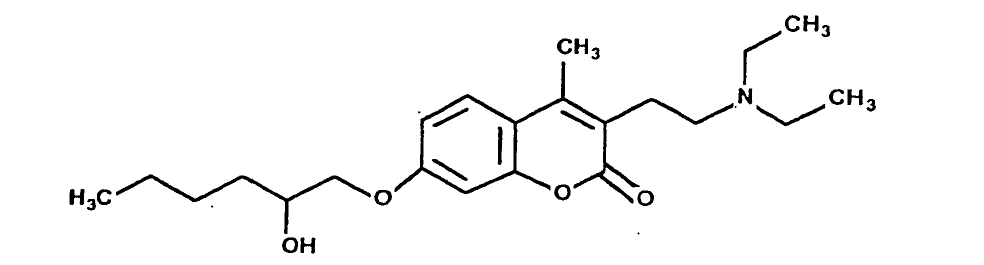 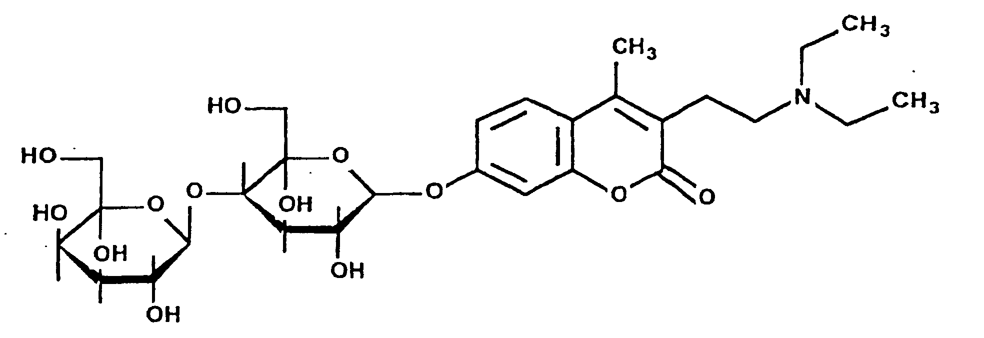 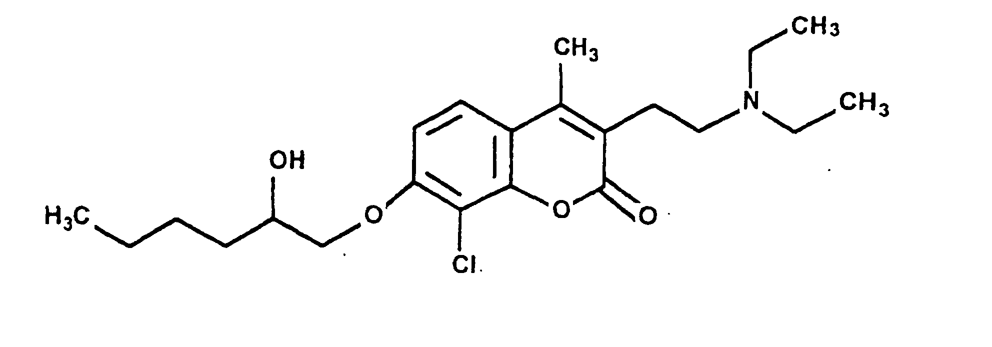 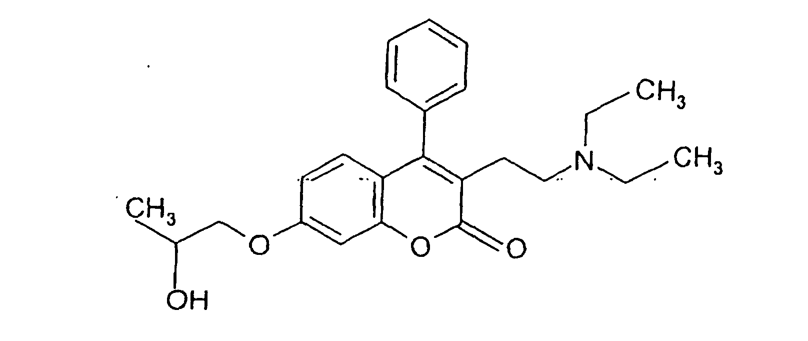 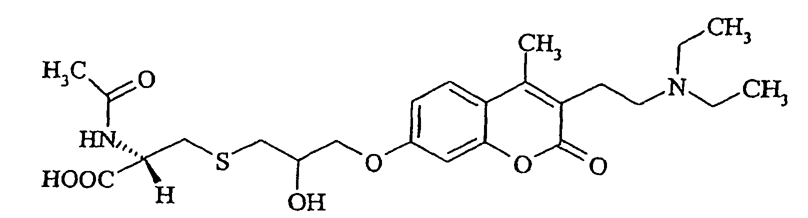 und deren Salze mit pharmazeutisch akzeptablen Säuren oder Basen.

2. Pharmazeutische Zusammensetzungen enthaltend ein Coumarin-Derivat gemäß Anspruch 1 in der Form von Kapseln, Tabletten, injizierbaren Lösungen, Sprays, Systemen zur kontrollierten Freisetzung, Cremes, Gelen und transdermalen Systemen.

3. Pharmazeutische Zusammensetzungen gemäß Anspruch 2 zur Behandlung von vaskulären (inklusive solchen, die der Freisetzung von Pro-Entzündungsmolekülen folgen), dermatologischen und allergischen Erkrankungen, von Hypercholesterolämie und von systemischen Infektionen.

4. Pharmazeutische Zusammensetzungen gemäß Anspruch 2 oder Anspruch 3 zur Behandlung von peripheren Gefäßkrankheiten, Anginaartigen Erkrankungen und zerebralen Gefäßkrankheiten, peripherer Ischämie und Ischämie von Organen.

5. Pharmazeutische Zusammensetzungen gemäß Anspruch 2 oder Anspruch 3 zur Behandlung von Thrombose und Bluthochdruck.

## Revendications

1. Dérivés de coumarine choisis dans le groupe constitué par : 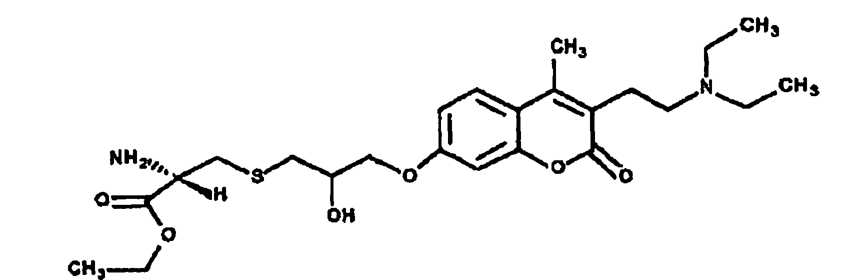 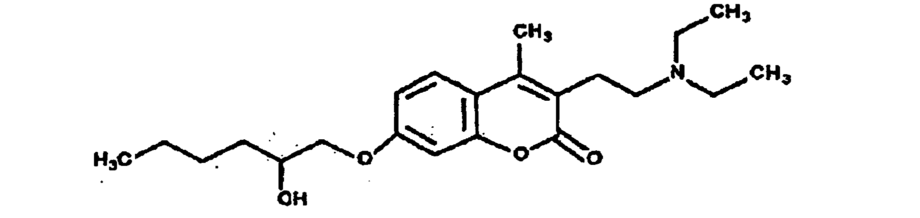 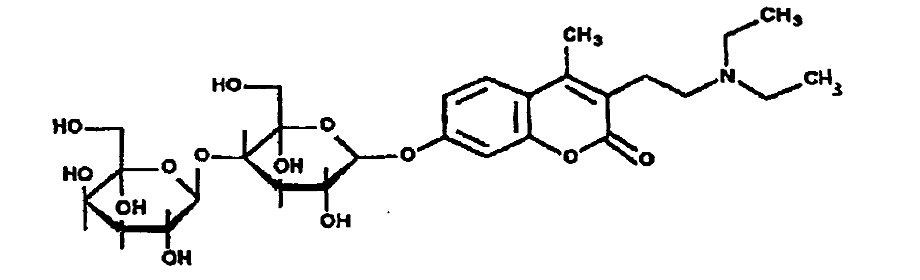 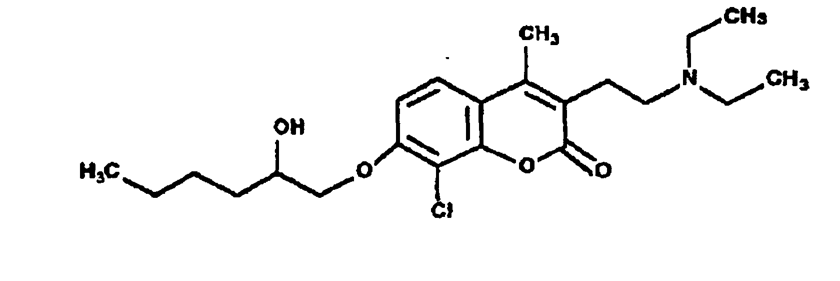 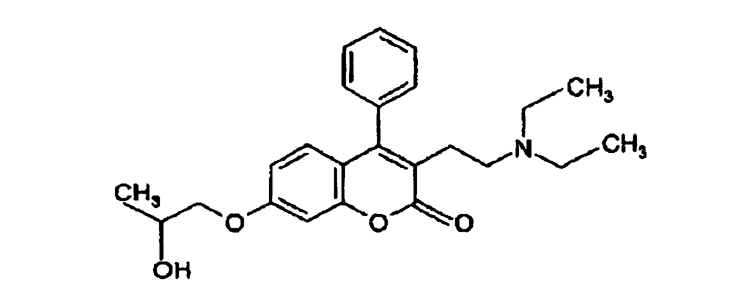 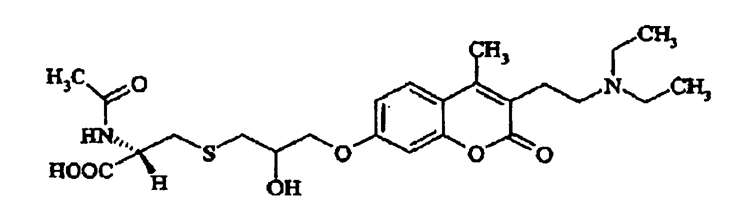 et les sels de ceux-ci avec des acides ou des bases pharmaceutiquement acceptables.

2. Compositions pharmaceutiques contenant un dérivé de coumarine tel que revendiqué dans la revendication 1, sous la forme de capsules, de comprimés, de solutions injectables, de pulvérisations, de systèmes à libération régulée, de crèmes, de gels et de systèmes transdermiques.

3. Compositions pharmaceutiques selon la revendication 2, pour le traitement de pathologies vasculaires (incluant celles découlant de la libération de molécules pro-inflammatoires), dermatologiques et allergiques, de l'hypercholestérolémie et d'infections systémiques.

4. Compositions pharmaceutiques selon les revendications 2 et 3, pour le traitement de vasculopathies périphériques, de troubles de type angine de poitrine et de vasculopathies cérébrales, d'une ischémie périphérique et d'une ischémie d'organes.

5. Compositions pharmaceutiques selon les revendications 2 et 3, pour le traitement d'une thrombose et de l'hypertension.
